# EUROPEAN PATENT SPECIFICATION

(11) **EP 2 566 579 B1**
(45) Date of publication and mention of the grant of the patent: **04.11.2015**
(21) Application number: 11723657.0
(22) Date of filing: 22.04.2011
(51) Int. Cl.: A61N 2/02, A61N 2/00, A61B 18/18

(54) **MEDICAL DEVICE FOR MAGNETOTHERAPY**
MEDIZINISCHE VORRICHTUNG FÜR MAGNETTHERAPIE
DISPOSITIF MÉDICAL POUR MAGNÉTOTHÉRAPIE

(30) Priority: 03.05.2010 IT PR20100040
(43) Date of publication of application: 13.03.2013
(73) Proprietor: F&B International S.r.l., 43121 Parma (IT)
(72) Inventor: GRAZIANO, Rossella, 20036 Meda (Milano) (IT); SESSA, Giulia, 43015 Noceto (Parma) (IT)
(74) Representative: Gotra, Stefano
(86) International application number: PCT/IB2011/051766
(87) International publication number: WO 2011/138702

(56) References cited:
- WO-A1-2008/109058
- WO-A2-2004/096343
- WO-A2-2008/005843
- WO-A2-2008/042902
- GB-A- 2 129 690
- US-A1- 2002 002 326
- US-A1- 2005 288 744
- US-A1- 2009 149 694
- US-A1- 2009 234 417
- US-B1- 6 978 179

## Description

### TECHNICAL FIELD AND BACKGROUND ART

The present invention relates to a medical device for magnetotherapy.

As is known, magnetotherapy is used in various medical fields, principally in physiotherapy. It is a treatment applied following prescription by specialists in orthopaedics, physiatry or geriatrics and consisting in suitable irradiation of a part of the human body with a magnetic field that is generally localized. Magnetotherapy has numerous fields of application and application modes, however it functions principally in the regularization of the electrochemical equilibrium of the cell and in restoring correct permeability of cell membrane. For that purpose, the areas affected by muscular, joint, bone and tissue pathologies are subjected to targeted irradiation treatments.

On the basis of the frequency of the electromagnetic field, it is possible to distinguish between high and low frequency magnetotherapy. High frequency magnetotherapy is based on the emission of waves with a carrier frequency greater than 15 MHz (preferably comprised between 20 MHz and 30 MHz) and a modulation frequency greater than 100 Hz (preferably comprised between 100 Hz and 5000 Hz). Low frequency magnetotherapy consists instead in generating electromagnetic fields with a frequency lower than 100 Hz.

Apparatus for performing high and low frequency magnetotherapy have already been marketed for several years now. Yet, the principle disadvantage of the known solutions lies in the unwieldiness of the apparatus, owing to the use of as many cables as there are applicators. In fact, the use of a plurality of applicators is foreseen, connected to the same power unit for the apparatus for magnetotherapy, in such a manner that different patients can have access to the same therapy at the same time.

For example, it is known from document WO2008/042902 a wrap (or a cradle) provided with coils for performing a single therapeutic program.

In this context, the technical task underlying this invention is to propose a medical device for magnetotherapy that overcomes the drawbacks of the prior art as cited hereinabove.

### DISCLOSURE OF INVENTION.

In particular, an aim of the present invention is to propose a medical device for magnetotherapy that is manageable and easily applicable to one or more patients.

The defined technical task and the specified aims are substantially achieved by a medical device for magnetotherapy, comprising the technical characteristics stated in one or more of the appended claims.

### BRIEF DESCRIPTION OF DRAWINGS

Further characteristics and advantages of the present invention will emerge more clearly from the indicative and thus non-limiting description of a preferred, but not exclusive, form of embodiment of a medical device for magnetotherapy, as illustrated in the appended drawings in which:
- figure 1 illustrates the block diagram of a medical device for magnetotherapy, according to the present invention;
- figure 2 illustrates a detail of the device in figure 1, in a partially sectioned perspective view;
- figure 3 illustrates some signals (low frequency signal, carrier and modulator of the high frequency signal) generated by the device in figure 1.

### BEST MODE FOR CARRYING OUT THE INVENTION.

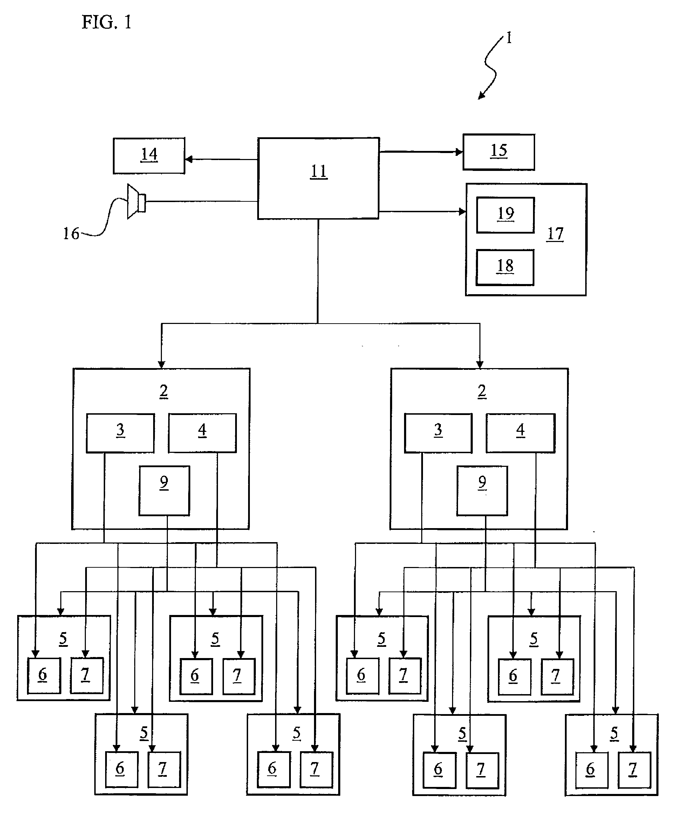

With reference to figure 1, a medical device for magnetotherapy has been indicated with 1. Originally, such medical device 1 comprises at least one therapeutic section 2 designed to generate a therapeutic program and made up of a first circuit 3 suitable for generating a high frequency signal and a second circuit 4 suitable for generating a low frequency signal LF.

The high frequency signal is a modulated signal having a carrier P with a frequency greater than 15 MHz and a modulator M with a frequency ranging between 100 Hz and 5000 Hz. Preferably, the carrier P has a frequency of 30 MHz. The frequencies of the modulator M can be modified according to an increasing or decreasing pattern on the basis of the therapeutic program. The low frequency signal LF has a frequency comprised between 6 Hz and 100 Hz.

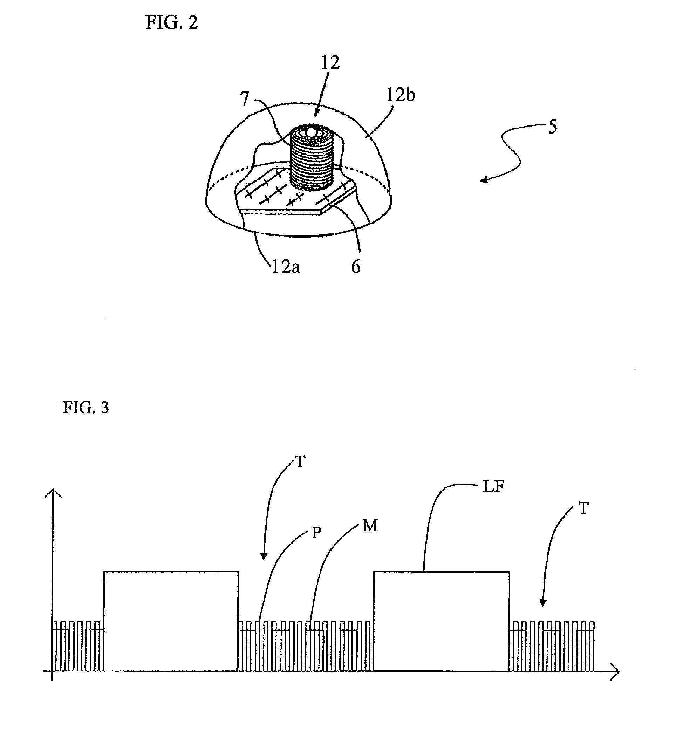

Each pulse of the low frequency signal LF alternates with a finite train T of pulses of the high frequency signal, as can be seen in figure 3. This alternation of signals is commonly known by the term "interpolation". The medical device 1 has at least one applicator 5 made up of an antenna 6 and a solenoid 7. In particular, the antenna 6 is powered (directly or indirectly) by said first circuit 3. The solenoid 7 is instead powered (directly or indirectly) by said second circuit 4. Preferably, the first circuit 3 and the second circuit 4 of the therapeutic section 2 are activated simultaneously so as to generate the corresponding therapeutic program. Alternatively, the first circuit 3 and the second circuit 4 of the therapeutic section 2 are activated individually.

Advantageously, the device 1 comprises an internal or external wireless transmitting module which transmits data to a wireless receiving module housed in the applicator 5. The data thereby received are used to bias the solenoid 7 and to power the antenna 6. Preferably, the medical device 1 comprises a plurality of therapeutic sections 2 designed to generate an equal number of therapeutic programs. In particular, each therapeutic section 2 is made up of a first circuit 3 and a second circuit 4. Preferably, the medical device 1 has a plurality of applicators 5, each of which is powered by one of said therapeutic sections 2. In particular, each applicator 5 is made up of an antenna 6 and a solenoid 7. The antenna 6 of each applicator 5 is powered (directly or indirectly) by one of said first circuits 3. The solenoid 7 of each applicator 5 is powered (directly or indirectly) by one of said second circuits 4.

Advantageously, a control unit 11 operatively active on the therapeutic sections 2 is provided so that they can be activated and managed in a mutually independent manner.

Each therapeutic section 2 is provided with a corresponding circuit 9 for regulation and control of the voltage and current, which proves to be operatively active on the solenoids 7 and on the antennas 6 of the applicators 5 powered by that therapeutic section 2 to vary the relative magnetic fields. In fact, for each solenoid 7, it is possible to regulate the current flow in the coil turns within a predetermined interval, so as to vary the value of the magnetic induction between 10 Gauss and 100 Gauss. Preferably, the circuit 9 for regulation and control of the voltage and current includes a stage for reversing the direction of circulation of the current. In particular, the applicators 5 powered by the corresponding therapeutic section 2 (that is, by the therapeutic section 2 to which said regulation circuit 9 belongs) are arranged in pairs in such a manner that in the two solenoids 7 of each pair of applicators 5, the current flows in opposite directions so that the magnetic fields generated are of opposite polarities. Preferably, the circuit 9 for regulation and control performs a feedback control operation on the current flowing in the corresponding solenoids 7.

In the embodiment illustrated in figure 1, the medical device 1 has two therapeutic sections 2, each one of which powers four applicators 5 arranged in two pairs. Therefore, it is possible to execute two distinct therapeutic programs, each of which can be applied simultaneously to four patients or to four different areas of an individual patient's body.

With reference to figure 2, each applicator 5 comprises a sheath 12 made up of a circular base 12a to which a spherical cap 12b is fastened. The antenna 6 is placed inside the sheath 12 and fastened to the circular base 12a. Above the antenna 6 there is the solenoid 7, the longitudinal axis of which is substantially perpendicular with respect to the circular base 12a. As mentioned hereinabove, at least one solenoid 7 is foreseen per applicator 5. Each applicator 5 may have more than one solenoid 7 arranged in sets, for example side by side to each other, for the purpose of developing opposite polarities simultaneously.

In alternative embodiments, the applicators 5 can be integrated in box bodies of various shapes or in bands for the body or in bed pads.

The control unit 11, preferably realized by means of a microprocessor, communicates with a keypad 14 and with a viewing screen 15 (or display). In particular, by means of the keypad 14, the patients set the therapeutic programs, while by means of the display 15 they view the information on the proper functioning of the medical device 1.

In an embodiment for blind patients, the device 1 is provided with a dedicated section 17 for blind patients, which is also managed by the control unit 11. In particular, the dedicated section 17 is made up of a first tactile surface 18 for setting the therapeutic programs and a second tactile surface 19 for providing information in Braille on the functioning of the medical device 1 (see figure 1). Preferably, the first tactile surface 18 consists in a tactile keypad shaped so as to fit the fingertips of a blind patient.

Preferably, the second tactile surface 19 consists in a pad of pins.

The possibility that the first tactile surface 18 coincides with the keypad 14 is further comprised. Preferably, the explanations are supplied by means of a voice indicator controlled by the control unit 11.

The medical device 1 can be powered through the mains supply or by battery. Preferably, the medical device 1 is powered by battery, whereas connection to the mains supply is used to recharge the battery. The battery charge status is monitored by the control unit 11 and in the case in which the residual charge is lower than the minimum predetermined level, the patient is notified of this by means of the display 15 or by means of a sound indicator 16. The interpolation is also managed by the control unit 11.

Communication between the therapeutic sections 2 and the applicators 5 is of the wireless type.

The operation of the medical device for magnetotherapy, according to the present invention is substantially the following.

The patient applies one of the applicators 5 to the body part to be treated and by means of the keypad 14 selects the therapeutic program to be carried out. In the event that the patient is blind, the selection of the therapeutic program is carried out using the first tactile surface 18 (which can coincide, as mentioned hereinabove, with said keypad 14). For example it is possible to carry out a therapeutic program to treat cephalalgia.

From the description provided hereinabove, the characteristics of the magnetotherapy medical device, according to the present invention, prove to be evident, as do the advantages.

In particular, owing to the fact that the applicators receive data from the device in the wireless mode, the device is very manageable and can be easily used also by a patient who is in a distant position from the relative therapeutic section. Consider, for example, a case in which several patients are using the same medical device for treatment simultaneously: they can situate themselves with their applicator in different treatment rooms, for the sake of convenience and privacy.

Furthermore, owing to the interpolation between the high frequency signal and the low frequency signal, the human body is able to assimilate better (that is, in a more effective manner) the selected therapeutic program.

Furthermore, owing to the presence of more than one therapeutic section designed to generate more than one therapeutic program, it is possible to perform such therapeutic programs simultaneously on more than one patient or on different areas of an individual patient's body.

Furthermore, owing to the timing carried out by the control unit, the therapeutic sections can be active simultaneously or it is possible to select some of them and activate them, while the other sections remain inactive.

Furthermore, owing to the circuit for regulation and control of the voltage and current, the magnetic induction of each solenoid can be changed in such a manner as to create the magnetic field suited to the pre-selected therapeutic program, achieving a dual polarity.

Furthermore, owing to the use of feedback control, the current flow in each solenoid is controlled more efficiently with respect to prior art solutions.

## Claims

1. Medical device (1) suitable for irradiating parts of the human body with a magnetic field for magnetotherapy treatment suitable for regularizating the electrochemical equilibrium of the cells and for restoring correct permeability of cell membranes, comprising:
a plurality of therapeutic sections (2) designed to generate an equal number of therapeutic programs, each of said therapeutic section (2) being made up of a first circuit (3) suitable for generating a high frequency signal and a second circuit (4) suitable for generating a low frequency signal (LF), said high frequency signal being a modulated signal having a carrier (P) with a frequency greater than 15 MHz and a modulator (M) with a frequency ranging between 100 Hz and 5000 Hz, said low frequency signal (LF) having a frequency comprised between 6 Hz and 100 Hz, each pulse of the low frequency signal (LF) alternating with a finite train (T) of pulses of said high frequency signal;
a plurality of applicators (5) to be applied to the body parts to be treated, each of said applicators (5) being powered by one of said therapeutic sections (2), each of said applicators (5) being made up of a solenoid (7) and an antenna (6), said antenna (6) being directly or indirectly powered by said first circuit (3) and said solenoid (7) being directly or indirectly powered by said second circuit (4), each therapeutic section (2) being provided with a corresponding circuit (9) for regulation and control of the voltage and current that is operatively active on the antennas (6) and solenoids (7) of the applicators (5) powered by that therapeutic section (2) in such a way as to vary the relative magnetic fields and thus the value of the current flowing in the solenoids (7), said circuit (9) for regulation and control including a stage for reversing the direction of circulation of the current flowing in the solenoids;
a wireless transmitting module which transmits data to a wireless receiving module housed in the applicator (5), the data received from said wireless receiving module being used to bias said solenoid (7) and/or to power said antenna (6).

2. Device (1) according to claim 1, wherein the communication between said therapeutic sections (2) and said applicators (5) is of the wireless type.

3. Device (1) according to claim 1 or 2, further comprising a control unit (11) operatively active on said therapeutic sections (2) so that they can be activated in a mutually independent manner.

4. Device (1) according to claim 1, wherein the first circuit (3) and the second circuit (4) of said at least one therapeutic section (2) are simultaneously activated.

5. Device (1) according to any of the preceding claims, wherein the value of the magnetic induction of said solenoid (7) varies between 10 Gauss and 100 Gauss.

6. Device (1) according to any of the preceding claims, further comprising a dedicated section (17) for blind users, said dedicated section (17) being made up of a first tactile surface (18) for setting the therapeutic programs and a second tactile surface (19) for providing information in Braille on the functioning of said medical device (1).

7. Device (1) according to any of the preceding claims, wherein each applicator (5) comprises a sheath (12) made up of a circular base (12a) to which a spherical cap (12b) is fastened, the corresponding antenna (6) being placed inside the sheath (12) and fastened to said circular base (12a), the corresponding solenoid (7) being placed above the antenna (6) with its longitudinal axis that is substantially perpendicular to the circular base (12a).

## Patentansprüche

1. Medizinische Vorrichtung (1), die geeignet ist, um Teile des menschlichen Körpers mit einem Magnetfeld für die Behandlung im Rahmen der Magnettherapie zu bestrahlen, geeignet, um das elektrochemische Zellgleichgewicht zu regulieren und die korrekte Permeabilität der Zellmembranen wiederherzustellen, umfassend:
eine Vielzahl therapeutischer Sektionen (2), die ausgestaltet sind, um eine gleiche Zahl an Therapieprogrammen zu generieren, wobei jede therapeutische Sektion (2) aus einem ersten Kreislauf (3) besteht, geeignet um ein Hochfrequenzsignal zu generieren, und einem zweiten Kreislauf (4), geeignet um ein Niederfrequenzsignal (LF) zu generieren, wobei es sich beim Hochfrequenzsignal um ein Modulationssignal handelt, aufweisend einen Träger (P) mit einer Frequenz von über 15 MHz und einen Modulator (M) mit einer Frequenz von 100 Hz bis 5000 Hz, wobei das Niederfrequenzsignal (LF) eine Frequenz von 6 Hz bis 100 Hz aufweist und jeder Impuls des Niederfrequenzsignals (LF) mit einer endlichen Folge (T) von Impulsen des Hochfrequenzsignals alterniert;
eine Vielzahl an Applikatoren (5), die an den zu behandelnden Körperteilen anzubringen sind, wobei ein jeder dieser Applikatoren (5) über eine der therapeutischen Sektionen (2) gespeist wird und ein jeder dieser Applikatoren (5) aus einem Solenoid (7) und einer Antenne (6) besteht, wobei die Antenne (6) unmittelbar oder mittelbar durch den ersten Kreislauf (3) gespeist wird und das Solenoid (7) unmittelbar oder mittelbar durch den zweiten Kreislauf (4) gespeist wird, wobei jede therapeutische Sektion (2) mit einem entsprechenden Kreislauf (9) versehen ist, um die Spannung und die Stromstärke zu regeln und zu steuern, die betriebswirksam auf den Antennen (6) und den Solenoiden (7) der Applikatoren (5), die über die betreffende therapeutische Sektion (2) gespeist werden, aktiv sind, sodass die entsprechenden Magnetfelder und somit der Wert des Stroms, der durch die Solenoide (7) fließt, variiert wird, wobei der Kreislauf (9) zum Regulieren und Steuern eine Phase umfasst, um die Zirkulationsrichtung des in den Solenoiden fließenden Stroms umzukehren;
ein drahtloses Übertragungsmodul, das Daten an ein drahtloses Empfängermodul, das im Applikator (5) untergebracht ist, überträgt, wobei die vom drahtlosen Empfängermodul empfangenen Daten genutzt werden, um das Solenoid (7) vorzumagnetisieren und/oder die Antenne (6) zu speisen.

2. Vorrichtung (1) nach Anspruch 1, wobei die Kommunikation zwischen den therapeutischen Sektionen (2) und den Applikatoren (5) drahtlos erfolgt.

3. Vorrichtung (1) nach Anspruch 1 oder 2, zudem umfassend eine Steuereinheit (11), die betriebswirksam auf die therapeutischen Sektionen (2) aktiv ist, sodass diese voneinander unabhängig aktiviert werden können.

4. Vorrichtung (1) nach Anspruch 1, wobei der erste Kreislauf (3) und der zweite Kreislauf (4) der mindestens einen therapeutischen Sektion (2) gleichzeitig aktiviert werden.

5. Vorrichtung (1) nach einem der vorhergehenden Ansprüche, wobei der Wert der magnetischen Induktion des Solenoids (7) zwischen 10 Gauss und 100 Gauss variiert.

6. Vorrichtung (1) nach einem der vorhergehenden Ansprüche, zudem umfassend eine spezielle Sektion (17) für blinde Nutzer, wobei diese spezielle Sektion (17) aus einer ersten taktilen Oberfläche (18) besteht, um die Therapieprogramme einzustellen, und aus einer zweiten taktilen Oberfläche (19), um Informationen in Brailleschrift über die Betriebsweise der medizinischen Vorrichtung (1) zu liefern.

7. Vorrichtung (1) nach einem der vorhergehenden Ansprüche, wobei jeder Applikator (5) eine Hülse (12) umfasst, die aus einer kreisförmigen Basis (12a) besteht, an der eine kugelförmige Kappe (12b) befestigt ist, wobei die entsprechende Antenne (6) in der Hülle (12) platziert und an der kreisförmigen Basis (12a) befestigt ist und das entsprechende Solenoid (7) über der Antenne (6) platziert ist, wobei dessen Längsachse im Wesentlichen rechtwinklig zur kreisförmigen Basis (12a) verläuft.

## Revendications

1. Dispositif médical (1) apte à irradier des parties du corps humain avec un champ magnétique pour un traitement par magnétothérapie destiné à régulariser l'équilibre électrochimique des cellules et à restaurer une bonne perméabilité des membranes cytoplasmiques, comprenant:
une pluralité de sections thérapeutiques (2) conçues pour générer un nombre égal de programmes thérapeutiques, chacune desdites sections thérapeutiques (2) étant constituée d'un premier circuit (3), apte à générer un signal haute fréquence, et d'un second circuit (4) apte à générer un signal basse fréquence (LF), ledit signal haute fréquence étant un signal modulé ayant une onde porteuse (P) dotée d'une fréquence supérieure à 15 MHz et un modulateur (M) doté d'une fréquence comprise entre 100 et 5000 Hz, ledit signal basse fréquence (LF) ayant une fréquence comprise entre 6 et 100 Hz, chaque impulsion du signal basse fréquence (LF) s'alternant avec une succession finie (T) d'impulsions dudit signal haute fréquence ;
une pluralité d'applicateurs (5) à appliquer aux parties du corps à traiter, chacun desdits applicateurs (5) étant alimenté par une desdites sections thérapeutiques (2), chacun desdits applicateurs (5) étant constitué d'un solénoïde (7) et d'une antenne (6), ladite antenne (6) étant directement ou indirectement alimentée par ledit premier circuit (3) et ledit solénoïde (7) étant directement ou indirectement alimentée par ledit second circuit (4), chaque section thérapeutique (2) étant pourvue d'un circuit correspondant (9) destiné à régler et à commander la tension et le courant étant fonctionnellement actifs sur les antennes (6) et les solénoïdes (7) des applicateurs (5) alimentés par cette section thérapeutique (2) de manière à varier les champs magnétiques correspondants et donc la valeur du courant circulant dans les solénoïdes (7), ledit circuit (9) de réglage et de commande incluant une étape destinée à inverser le sens de circulation du courant circulant dans les solénoïdes ;
un module de transmission sans fil qui transmet des données à un module de réception sans fil logé dans l'applicateur (5), les données reçues dudit module de réception sans fil étant utilisées pour polariser ledit solénoïde (7) et/ou pour alimenter ladite antenne (6).

2. Dispositif (1) selon la revendication 1, dans lequel la communication entre lesdites sections thérapeutiques (2) et lesdits applicateurs (5) est de type sans fil.

3. Dispositif (1) selon les revendications 1 ou 2, comprenant de plus une unité de commande (11) fonctionnellement active sur lesdites sections thérapeutiques (2) de sorte qu'elles puissent être activées d'une façon mutuellement indépendante.

4. Dispositif (1) selon la revendication 1, dans lequel le premier circuit (3) et le second circuit (4) de ladite au moins une section thérapeutique (2) sont activés simultanément.

5. Dispositif (1) selon l'une quelconque des revendications précédentes, dans lequel la valeur de l'induction magnétique dudit solénoïde (7) varie entre 10 et 100 Gauss.

6. Dispositif (1) selon l'une quelconque des revendications précédentes, comprenant de plus une section dédiée (17) aux utilisateurs aveugles, ladite section dédiée (17) étant composée d'une première surface tactile (18) pour établir les programmes thérapeutiques et d'une seconde surface tactile (19) destinée à fournir des informations en braille sur le fonctionnement dudit dispositif médical (1).

7. Dispositif (1) selon l'une quelconque des revendications précédentes, dans lequel chaque applicateur (5) comprend une gaine (12) composée d'une base circulaire (12a) à laquelle une calotte sphérique (12b) est attachée, l'antenne correspondante (6) étant placée à l'intérieur de la gaine (12) et attachée à ladite base circulaire (12a), le solénoïde correspondant (7) étant placé au-dessus de l'antenne (6) avec son axe longitudinal étant substantiellement perpendiculaire à la base circulaire (12a).
